# EUROPEAN PATENT APPLICATION

(11) **EP 3 278 776 A1**
(43) Date of publication of application: **07.02.2018**
(21) Application number: 16182262.2
(22) Date of filing: 01.08.2016
(51) Int. Cl.: A61F 7/00, F16F 15/00

(54) **FLUID WARMING APPARATUS**

(71) Applicant: The Surgical Company International B.V., 3821 AH Amersfoort (NL)
(72) Inventor: The designation of the inventor has not yet been filed
(74) Representative: Hindles Limited

(57) **Abstract**

The invention provides forced air warming apparatus for providing air to a patient temperature regulation tool for regulating the temperature of a patient, the forced air warming apparatus comprising: a housing; an air pressurising device provided within the housing, the air pressurising device being configured to receive an air intake and to pressurise air from the air intake; and a suspension configured to restrict transfer to the housing of vibrations caused by the air pressurising device pressurising the said air from the air intake.

## Description

### Field of the invention

The invention relates to fluid (e.g. forced air) warming apparatus; a patient temperature regulation system comprising fluid (e.g. forced air) warming apparatus; a method of pressurising air; and a method of regulating the temperature of a (typically human) patient.

### Background to the invention

Devices used for forced air warming typically consist of a fan and heater to create a stream of warm air. The warm air is applied to a patient via a hose and a perforated blanket which is draped over a patient's body. Openings in the blanket cause the warm air to exit the blanket at the patient side, thereby heating the patient by means of forced convection.

For effective heating of a patient, a volumetric flowrate of approximately 0.025 m³/s at a temperature of 45°C is typically needed. Fans which can generate this flowrate typically generate uncomfortable vibrations and noise. This makes it impractical to mount forced air warming devices close to a patient's head, or to a patient's bed. This in turn means that forced air warming devices are provided remote from the patient, which increases the length of the hose required to carry the air flow from the forced air warming device to the perforated blanket. This reduces thermal efficiency and can cause inconvenience in having to safely route the hose between the forced air warming device and the blanket.

Accordingly, new ways to improve the thermal efficiency of forced air warming systems are required. In addition, new ways to more conveniently arrange forced air warming systems would also be beneficial.

### Summary of the invention

A first aspect of the invention provides fluid (e.g. forced air) warming apparatus (e.g. a forced air warmer) for providing (typically heated, typically pressurised) fluid (typically air, typically an air flow) to a (typically convective) patient temperature regulation tool (such as a fluid warming blanket or mattress) for regulating the temperature of a (typically human) patient (typically during, before or after a medical procedure or surgery). Typically the fluid warming apparatus comprises a (typically external, typically apparatus) housing. Typically the fluid warming apparatus further comprises a fluid (e.g. air) pressurising device (e.g. a fan) provided within the housing. Typically the fluid (e.g. air) pressurising device is configured to receive a fluid (e.g. air) intake (e.g. an ambient air intake). Typically the fluid (e.g. air) pressurising device is further configured to pressurise fluid (typically air) from the fluid (e.g. air) intake (e.g. when the fluid (e.g. air) pressurising device is a fan, by rotation of one or more blades of the said fan about an axis). Typically the fluid warming apparatus further comprises a (e.g. resilient) suspension configured to restrict transfer to the housing of vibrations (typically from within the housing), typically caused by (e.g. operation of) the fluid (e.g. air) pressurising device pressurising the said fluid (e.g. air) from the fluid (e.g. air) intake (in use).

The reduction in vibrations transferred to the housing allows the fluid (e.g. forced air) warming apparatus to be mounted closer to the patient (e.g. closer to the patient's head), and even on the patient's bed frame, without transmitting a significant amount of vibration (and therefore without causing associated discomfort) to the patient. This increases the comfort of the patient and the convenience of the fluid warming apparatus, particularly in surgical environments in which space is limited. In addition, as the fluid warming apparatus can be provided closer to the patient, a shorter hose can be used to carry air from the fluid warming apparatus to the patient temperature regulation tool, which improves thermal efficiency and the comfort and safety of the surgical environment.

Typically the fluid (e.g. air) pressurising device comprises a movable portion (e.g. one or more rotatable blades) and a static (relative to the moveable portion) portion (e.g. a fluid (e.g. air) pressurising device housing), the movable portion being moveable relative to the static portion to thereby pressurise fluid (e.g. air) from the fluid (e.g. air) intake.

Operation of the fluid (e.g. air) pressurising device typically causes vibration of the fluid (e.g. air) pressurising device itself by virtue of its movement (e.g. vibration of the static portion is typically caused by movement of the moveable portion relative to the static portion). Accordingly, typically the fluid (e.g. air) pressurising device is configured to vibrate in use.

Typically the fluid (e.g. air) pressurising device is mechanically coupled to the housing by way of the said suspension. Typically the said suspension enables (e.g. vibrational) movement of the fluid (e.g. air) pressurising device relative to the housing to thereby restrict transfer of vibrations from the fluid (e.g. air) pressurising device to the housing (e.g. when the fluid (e.g. air) pressurising device is a fan, vibrations caused by rotation of a rotor of a motor of the fan and/or rotation of the blades of the fan). By mechanically coupling the fluid (e.g. air) pressurising device to the housing by way of a suspension which enables (e.g. vibrational) movement of the fluid (e.g. air) pressurising device relative to the housing, vibration of, and unwanted acoustic noise transmitted by, the housing is reduced.

It may be that the static portion of the fluid pressurising device is coupled to the housing by way of the said suspension, the said suspension enabling (e.g. vibrational) movement of the said static portion relative to the housing to thereby restrict transfer of vibrations from the said static portion to the said housing.

Typically the fluid pressurising device is an air pressurising device configured to generate pressurised fluid (e.g. air).

Typically the fluid (e.g. air) pressurising device is configured to generate pressurised air in a pressure chamber provided within the housing (the pressure chamber typically being separate from the housing, the pressure chamber typically being discrete from the housing). Typically the pressure chamber is encapsulated by the housing. By generating the pressurised fluid in a pressure chamber, the transfer to the housing (and thus the external environment) of acoustic waves caused by the (typically turbulently flowing) pressurised fluid is reduced.

Operation of the fluid (e.g. air) pressurising device typically causes vibrations of the pressure chamber by virtue of the (typically turbulent) pressurised fluid (e.g. air) it generates engaging the pressure chamber, thereby causing said vibrations. In addition, vibrations of the fluid (e.g. air) pressurising device may be transferred to the pressure chamber.

It may be that the pressure chamber is mechanically coupled to the housing by way of the said suspension to thereby restrict transfer of vibrations from the pressure chamber to the housing. By mechanically coupling the pressure chamber to the housing by the said suspension, transfer to the housing of unwanted vibrations of the pressure chamber caused by turbulence of pressurised fluid (e.g. air) therein is thereby restricted (i.e. by the pressure chamber). In addition, transfer of any vibration of the fluid (e.g. air) pressurising device to the housing by way of the pressure chamber is restricted.

It may be that the fluid (e.g. air) pressurising device is mechanically coupled to the housing by way of the pressure chamber.

It may be that the fluid (e.g. air) pressurising device is mechanically coupled to the housing by way of the suspension by virtue of the fact that the fluid (e.g. air) pressurising device is (e.g. rigidly) coupled to the pressure chamber and the pressure chamber is coupled to the housing by way of the suspension.

It may be that the fluid (e.g. air) pressurising device is rigidly coupled to the pressure chamber.

It may be that the fluid (e.g. air) pressurising device is mechanically coupled to the housing by way of the suspension by virtue of the fact that the fluid (e.g. air) pressurising device is coupled to the pressure chamber by way of the said suspension and the pressure chamber is (e.g. rigidly) coupled to the housing. It may be that the pressure chamber is rigidly coupled to the housing.

In some cases, the fluid (e.g. air) pressurising device is coupled to the pressure chamber by a first (e.g. resilient) suspension enabling relative (e.g. vibrational) movement of the fluid (e.g. air) pressurising device and the pressure chamber to thereby restrict transfer of vibrations of the fluid (e.g. air) pressurising device to the pressure chamber, and the pressure chamber is coupled to the housing by a second (e.g. resilient) suspension enabling relative (e.g. vibrational) movement of the pressure chamber and the housing to thereby restrict transfer of vibrations of the pressure chamber to the housing. It may be that the first and second suspensions are independent of each other.

It may be that the pressure chamber is not rigidly connected to the housing. It may be that the fluid (e.g. air) pressurising device is not rigidly connected to the pressure chamber. Typically the fluid (e.g. air) pressurising device is not rigidly connected to the housing.

Typically a volume between the housing and the pressure chamber remains unpressurised in use. For example, the volume between the housing and the pressure chamber may be configured to remain at atmospheric pressure or below in use.

Typically the said suspension is provided within (e.g. encapsulated by) the housing.

Typically the fluid (e.g. air) pressurising device comprises a fan. Typically the fluid (e.g. air) pressurising device (e.g. a fan) comprises one or more (typically a plurality of) blades rotatable about an axis of rotation to thereby generate the said pressurised fluid (e.g. air) from the fluid (e.g. air) intake. Typically at least a portion of each of the said one or more blades is provided within the pressure chamber (i.e. in contact with pressurised air in the pressure chamber). It may be that the said one or more blades is encapsulated within the pressure chamber.

By providing the said blades within (e.g. by encapsulating the said one or more blades within) the pressure chamber, transfer to the housing of acoustic waves generated by rotation of the blades is restricted (e.g. said acoustic waves being attenuated, e.g. absorbed or reflected, by the pressure chamber), thereby further reducing the acoustic noise transmitted by the housing.

Typically the fluid (e.g. air) pressurising device (e.g. the fan) comprises a motor. Typically the blades (where provided) are rotatable by the said motor. It may be that the fluid (e.g. air) pressurising device (e.g. the fan) comprises a hub which defines the axis of rotation about which the blades are configured to rotate. It may be that the hub comprises (e.g. houses) the motor (or at least a portion of a rotor thereof).

Typically the motor (or at least a rotor of the motor) is mechanically coupled to the blades.

Typically the motor is provided within or is surrounded by the pressure chamber.

It may be that the motor is coupled to the pressure chamber or to housing by the said suspension which enables (e.g. vibrational) movement of the motor relative to the pressure chamber or the housing (as the case may be) to thereby restrict transfer of vibrations from the motor to the housing.

It may be that the pressure chamber is annular.

It may be that the hub is provided within an annulus of the annular pressure chamber.

It may be that the motor (or at least a portion of a rotor thereof) is provided within an annulus of the annular pressure chamber.

It may be that the axis of rotation of the blades of the fan extends through an annulus of the pressure chamber.

It may be that the pressure chamber has a spiral shape.

It may be that the pressure chamber has a (typically annular, typically spiral) scroll shape.

It may be that the pressure chamber comprises a (e.g. annular) volute. It may be that the fluid (e.g. air) pressurising device and the pressure chamber together comprise a (e.g. annular) volute fluid (e.g. air) pump.

Typically the housing comprises a fluid (e.g. air) inlet port.

Typically the fluid (e.g. air) pressurising device is configured to receive the said fluid (e.g. air) intake by way of the said fluid (e.g. air) inlet port of the housing.

Typically the pressure chamber comprises a fluid (e.g. air) inlet port. It may be that the fluid (e.g. air) inlet port of the pressure chamber is provided in an annulus of the said pressure chamber.

Typically the fluid (e.g. air) pressurising device is configured to receive the said fluid (e.g. air) intake by way of the said fluid (e.g. air) inlet port of the pressure chamber.

Typically the fluid (e.g. air) pressurising device is configured to receive the said fluid (e.g. air) intake by way of the said fluid (e.g. air) inlet ports of both the housing and the pressure chamber.

Typically the pressure chamber comprises a fluid (e.g. air) outlet port. It may be that the fluid (e.g. air) outlet port is an outlet port of a or the (typically annular) volute. In this case, the fluid (e.g. air) outlet port of the pressure chamber is typically provided at an end of the volute remote from the fluid (e.g. air) intake of the pressure chamber (and typically a fluid (e.g. air) intake of the volute).

Typically the housing comprises a fluid (e.g. air) outlet port.

It may be that the pressure chamber comprises a fluid (e.g. air) outlet port and the housing comprises a fluid (e.g. air) outlet port, wherein the fluid (e.g. air) outlet port of the pressure chamber is in fluid communication with the fluid (e.g. air) outlet port of the housing by way of a (first) coupler. Typically the (first) coupler enables (e.g. vibrational) movement of the pressure chamber relative to the housing to thereby restrict transfer of vibrations from the pressure chamber to the housing.

Typically at least a portion of the (first) coupler is resilient (e.g. elastic). Typically at least a portion of the (first) coupler is resiliently (e.g. elastically) deformable. It may be that the (first) coupler comprises an elastic sleeve comprising a first end coupled to the outlet port of the pressure chamber and a second end opposite the first end coupled to an outlet port of the housing.

It may be that the fluid warming apparatus comprises a hose connector (e.g. for connecting a hose to the housing and configured to receive pressurised fluid (e.g. air) from the fluid (e.g. air) pressurising device, e.g. by way of the pressure chamber) coupled to the housing by way of a (second) coupler which enables (e.g. vibrational) movement of the hose connector relative to the housing to thereby restrict transfer of vibrations from the hose connector to the housing.

It may be that at least a portion of the (second) coupler is resilient (e.g. elastic). It may be that at least a portion of the (second) coupler is resiliently (e.g. elastically) deformable. It may be that the (second) coupler comprises an elastic sleeve comprising a first portion coupled to (e.g. the outlet port of) the housing and a second portion coupled to the hose connector. It may be that at least a portion of the (second) coupler is provided in the outlet port of the housing. It may be that a portion of the (second) coupler surrounds the said hose connector in a plane perpendicular to a longitudinal axis of the hose connector.

Typically at least a portion of the (or each) suspension is configured (e.g. comprises one or more dampers configured) to damp (e.g. vibrational) movement of the fluid (e.g. air) pressurising device relative to the housing. It may be that at least a portion of the (or the first) suspension is (e.g. the or the first suspension may comprise one or more dampers) configured to damp (e.g. vibrational) movement of the fluid (e.g. air) pressurising device relative to the pressure chamber. It may be that at least a portion of the (or the second) suspension is (e.g. the or the second suspension may comprise one or more dampers) configured to damp (e.g. vibrational) movement of the pressure chamber relative to the housing. Typically the said damping portion is configured to dissipate energy from the said relative (e.g. vibrational) movement to thereby restrict transfer of vibrations to the housing.

It may be that at least a portion of the (or each said) suspension is resilient (e.g. elastic).

It may be that at least a portion of the (or each said) suspension is resiliently (e.g. elastically) deformable (e.g. by movement of the fluid pressurising device relative to the housing and/or by movement of the fluid pressurising device relative to the pressure chamber and/or by movement of the pressure chamber relative to the housing) to thereby restrict transfer of said vibrations to the housing.

It may be that at least a portion of the (or each said) suspension comprises an elastomer (e.g. a resiliently deformable elastomer).

It may be that at least a portion of the (or each said) suspension is configured to store energy (e.g. from (e.g. vibrational) movement of the fluid pressurising device relative to the housing and/or from (e.g. vibrational) movement of the fluid pressurising device relative to the pressure chamber and/or from (e.g. vibrational) movement of the pressure chamber relative to the housing) to thereby restrict transfer of said vibrations to the housing.

It may be that the (or each said) suspension comprises one or more springs configured to restrict transfer of said vibrations to the housing. Typically the said one or more springs are configured to store energy (e.g. from (e.g. vibrational) movement of the fluid pressurising device relative to the housing and/or from movement of the fluid pressurising device relative to the pressure chamber and/or from movement of the pressure chamber relative to the housing) to thereby restrict transfer of said vibrations to the housing.

It may be that the (or each) suspension comprises one or more shock absorbers configured to restrict transfer of said vibrations to the housing. The suspension may comprise one or more shock absorbers between the fluid pressurising device and the housing. The (or the first) suspension may comprise one or more shock absorbers between the fluid pressurising device and the pressure chamber. The (or the second) suspension may comprise one or more shock absorbers provided between the housing and the pressure chamber.

It may be that the (or each said) suspension comprises one or more (e.g. material) hinges (e.g. enabling (e.g. vibrational) movement of the fluid pressurising device relative to the housing and/or enabling movement of the fluid pressurising device relative to the pressure chamber and/or movement of the pressure chamber relative to the housing) configured to restrict transfer of said vibrations to the housing.

It may be that the (or each said) suspension comprises a plurality of (e.g. discrete) suspension mountings (e.g. enabling (e.g. vibrational) movement of the fluid pressurising device relative to the housing and/or enabling movement of the fluid pressurising device relative to the pressure chamber and/or movement of the pressure chamber relative to the housing) configured to restrict transfer of said vibrations to the housing.

It may be that each of the said suspension mountings mechanically couples the fluid (e.g. air) pressurising device to the housing (e.g. by coupling the fluid pressurising device to the pressure chamber or coupling the pressure chamber to the housing). One or more or each of the suspension mountings may have any feature or any combination of features of the suspension described herein.

It may be the suspension mountings of the (or each said) said suspension are identical to each other. Alternatively, one or more said suspension mountings of the or each said suspension may be different from one or more other said suspension mountings.

It may be that the suspension mountings of the (or each said) said suspension are spaced from each other. It may be that the suspension mountings of the (or each said) said suspension are spaced from each other about the axis of rotation of the fluid (e.g. air) pressurising device. It may be that the suspension mountings of the (or each) said suspension are spaced from each other about a (typically outer) perimeter of the fluid (e.g. air) pressurising device or a (typically outer) perimeter of the pressure chamber. It may be that the suspension mountings of the (or each) said suspension extend from an outer perimeter of the pressure chamber. It may be that the suspension mountings of the (or each) said suspension are uniformly spaced from each other. The suspension mountings may be distributed symmetrically about a centre of gravity of the fluid (e.g. air) pressurising device and/or a centre of gravity of the pressure chamber (and/or a centre of gravity of the combination of the pressure chamber and the fluid (e.g. air) pressurising device). This helps to distribute load evenly among the suspension mountings.

Typically the suspension mountings are provided in the same (typically vertical) plane (as each other). Providing the suspension mountings in the same plane as each other helps to isolate the vibrations of the fluid (e.g. air) pressurising device from the housing.

It may be that the said plane comprises (i.e. is aligned with) a centre of gravity of the fluid (e.g. air) pressurising device and/or a centre of gravity of the pressure chamber (where provided) and/or a centre of gravity of a combination of the pressure chamber and the fluid (e.g. air) pressurising device. Providing the suspension mountings in the same plane as the said centre of gravity also helps to limit and control the three dimensional vibrations of the fluid (e.g. air) pressurising device and/or the pressure chamber.

It may be that one or more or each of the suspension mountings of the (or each) said suspension comprises one or more of the group comprising: a damper; a resilient element; a resiliently deformable element; an elastic element; an elastically deformable element; an energy storage element; an elastomeric element; a spring; and a hinge (e.g. a material hinge).

It may be that an axis of rotation of the fluid (e.g. air) pressurising device (e.g. the blades of the fan) extends horizontally (i.e. the fluid (e.g. air) pressurising device (e.g. the fan) is vertically mounted). Alternatively it may be that the axis of rotation of the fluid (e.g. air) pressurising device extends vertically (i.e. the fluid (e.g. air) pressurising device is horizontally mounted). The term vertical is to be construed as being parallel to a local gravitational field (e.g. the earth's gravitational field in the environment comprising the fluid warming apparatus). The term horizontal is to be construed as being perpendicular to the vertical.

It may be that the fluid (e.g. air) pressurising device is a centrifugal fan, but this is not necessarily the case. For example, the fluid (e.g. air) pressurising device may be an axial fan, or any other suitable device for pressurising fluid (e.g. air).

Typically the housing is exposed to the environment.

Typically the fluid warming apparatus is configured to output heated fluid (e.g. air) having a volumetric flow rate of at least 0.001 m3/s, more typically at least 0.002m³/s, even more typically at least 0.0025m³/s, in some cases at least 0.01 m3/s, in some cases at least 0.02m³/s, in some cases at least 0.025m³/s.

Typically the fluid warming apparatus further comprises a heater configured to heat fluid (e.g. air) from the fluid (e.g. air) intake. Typically the heater is provided in a fluid (e.g. air) flow path extending between the fluid (e.g. air) intake of the housing and a or the outlet of the housing. It may be that the heater is provided within the pressure chamber (e.g. adjacent to the fluid (e.g. air) outlet of the pressure chamber). Alternatively, the heater may be provided in the housing (e.g. between the said housing and the said pressure chamber). For example, the heater may be provided adjacent to the fluid (e.g. air) intake of the housing or adjacent to the hose connector (where provided) or adjacent to the outlet of the housing.

Typically the fluid warming apparatus is configured to output heated fluid (e.g. air) having a temperature of at least 30°C, more typically at least 35°C, more typically at least 40°C, typically less than 55°C, preferably between 42°C and 48°C, more preferably between 44°C and 46°C, most preferably at 45°C.

It may be that the housing is provided with a mounting (e.g. one or more feet) by which the housing can be mounted to a mount (e.g. mounting surface). It may be that the mounting (e.g. feet) is resilient so as to enable (e.g. vibrational) movement of the housing relative to the mount to thereby restrict transfer of vibrations from the housing to the mount. For example, the said mounting (e.g. one or more feet) may be resiliently deformable. It may be that the said mounting (e.g. one or more feet) comprises elastomeric material.

A second aspect of the invention provides a patient temperature regulation system comprising: fluid (e.g. forced air) warming apparatus according to the first aspect of the invention. The patient temperature regulation system typically further comprises a patient temperature regulation tool configured to receive heated fluid (e.g. air) from the fluid (e.g. forced air) warming apparatus, typically by way of a hose.

It may be that the fluid warming apparatus is provided adjacent to a (typically human) patient (e.g. adjacent to a patient's head) or mounted to a bed frame (the bed frame typically carrying a human patient).

It may be that (e.g. the housing of) the said fluid warming apparatus is mechanically coupled to the said bed frame by a resilient mounting enabling relative (e.g. vibrational) movement of the fluid warming apparatus and the bed frame to thereby restrict transfer of vibrations from (e.g. the housing of) the fluid warming apparatus to the bed frame.

Typically the patient temperature regulation tool is configured to heat a patient by convection using the heated fluid (e.g. air) received from the fluid warming apparatus.

It may be that the patient temperature regulation tool comprises a blanket, a garment or an underbody (e.g. mattress) configured to regulate the temperature of the patient by forced convection. It may be that the patient temperature regulation tool comprises a pressure chamber having a fluid (e.g. air) impermeable surface on a first side remote from the patient and a fluid (e.g. air) permeable surface on a second side opposite the first side and facing the patient. It may be that the fluid (e.g. air) impermeable surface has a port extending therethrough and configured to receive heated, pressurised fluid (e.g. air) from the fluid warming apparatus. It may be that the fluid (e.g. air) permeable surface allows the passage of fluid (e.g. air) therethrough onto the patient (e.g. by way of openings or perforations).

It may be that the patient temperature regulation tool comprises a perforated blanket which is (or which is configured to be) draped over the patient's body. Typically the blanket comprises openings by way of which received heated fluid (e.g. air) exits the blanket to thereby heat the patient by means of forced convection.

Typically the hose configured to carry fluid (e.g. air) from the pressure chamber to the patient temperature regulation tool is flexible.

A third aspect of the invention provides a method of pressurising fluid (e.g. air) in a fluid warming apparatus for providing fluid (typically air, typically an air flow) to a (typically convective) patient temperature regulation tool for regulating the temperature of a (typically human) patient (typically during, before or after a medical procedure or surgery), the method comprising: providing a fluid (e.g. air) pressurising device within (e.g. encapsulated by) a (typically external, typically apparatus) housing. Typically the method further comprises the fluid (e.g. air) pressurising device receiving a fluid (e.g. air) intake. Typically the method further comprises the fluid (e.g. air) pressurising device pressurising fluid (typically generating higher pressure air, typically a pressurised air flow) from the fluid (e.g. air) intake (typically by rotation of one or more blades of a fan provided within the housing about an axis of rotation), typically thereby causing vibrations (typically within the housing). Typically the method further comprises a suspension restricting transfer of (typically said) vibrations (typically from within the housing) to the housing (e.g. by moving and/or resiliently deforming the said suspension).

Typically the method comprises the suspension restricting transfer of said vibrations to the housing by enabling (e.g. vibrational) movement of the fluid (e.g. air) pressurising device relative to the housing to thereby restrict transfer of vibrations from the fluid (e.g. air) pressurising device to the housing.

Typically the fluid pressurising device is an air pressurising device (e.g. a fan). Typically the fluid intake comprises an air intake. Typically the said pressurised fluid comprises pressurised air. Typically the method comprises pressurising air from the air intake by rotation of one or more blades of the fluid (e.g. air) pressurising device about an axis of rotation.

Typically the method comprises the fluid (e.g. air) pressurising device pressurising fluid (e.g. air) from the fluid (e.g. air) intake in a pressure chamber provided within the housing. Typically the pressure chamber is encapsulated by the housing.

Typically the fluid (e.g. air) pressurising device is coupled to the housing by way of the pressure chamber.

It may be that the method comprises the said suspension restricting transfer of said vibrations from the fluid (e.g. air) pressurising device to the housing by enabling (e.g. vibrational) movement of the fluid (e.g. air) pressurising device relative to the pressure chamber (e.g. which suspension mechanically couples the fluid (e.g. air) pressurising device to the housing). It may be that the pressure chamber is rigidly coupled to the housing.

It may be that the method comprises the said suspension restricting transfer of vibrations from the fluid (e.g. air) pressurising device to the housing by enabling (e.g. vibrational) movement of the pressure chamber relative to the housing (e.g. which suspension mechanically couples the pressure chamber to the housing). It may be that the fluid (e.g. air) pressurising device is rigidly coupled to the pressure chamber.

It may be that the method comprises the said suspension restricting transfer of vibrations from the pressure chamber to the housing by enabling (e.g. vibrational) movement of the pressure chamber relative to the housing (e.g. which suspension mechanically couples the pressure chamber to the housing).

It may be that the method comprises a first suspension restricting transfer of said vibrations from the fluid (e.g. air) pressurising device to the housing by enabling (e.g. vibrational) movement of the fluid (e.g. air) pressurising device relative to the pressure chamber (e.g. which first suspension mechanically couples the fluid (e.g. air) pressurising device to the housing) and a second suspension restricting transfer of said vibrations from the pressure chamber to the housing by enabling (e.g. vibrational) movement of the pressure chamber relative to the housing (e.g. which second suspension mechanically couples the pressure chamber to the housing).

The method may further comprise providing one or more blades of the fluid (e.g. air) pressurising device (e.g. encapsulating the said one or more blades of the fluid (e.g. air) pressurising device) within the said pressure chamber.

The method may comprise the pressure chamber attenuating (e.g. absorbing or reflecting) acoustic waves generated by the rotation of the said one or more blades of the fluid (e.g. air) pressurising device to thereby reduce acoustic noise transmitted by the housing (e.g. to the external environment).

It may be that the method comprises receiving the said fluid (e.g. air) intake by way of a fluid (e.g. air) inlet port of the housing.

It may be that the method comprises receiving the said fluid (e.g. air) intake by way of a fluid (e.g. air) inlet port of the pressure chamber.

It may be that the method comprises receiving the said fluid (e.g. air) intake by way of fluid (e.g. air) inlet ports of both the housing and the pressure chamber.

It may be that the pressure chamber comprises a fluid (e.g. air) outlet port and the housing comprises a fluid (e.g. air) outlet port. It may be that the method comprises fluidly coupling the fluid (e.g. air) outlet port of the pressure chamber to the fluid (e.g. air) outlet port of the housing by way of a (first) coupler. Typically the method comprises, by way of the (first) coupler, moving the pressure chamber relative to the housing to thereby restrict transfer of vibrations from the pressure chamber to the housing.

Typically at least a portion of the (first) coupler is resilient (e.g. elastic). It may be that the method comprises resiliently (e.g. elastically) deforming at least a portion of the (first) coupler to thereby restrict transfer of vibrations from the fluid (e.g. air) pressurising device to the housing. It may be that the (first) coupler comprises an elastic sleeve comprising a first end coupled to the outlet port of the pressure chamber and a second end opposite the first end coupled to an outlet port of the housing. It may be that the method comprises elastically deforming the elastic sleeve to thereby restrict transfer of vibrations from the fluid (e.g. air) pressurising device to the housing.

It may be that the fluid warming apparatus comprises a hose connector for coupling a (typically flexible) hose to the outlet port of the housing. It may be that the method comprises coupling a (typically flexible) hose to the outlet port of the housing by way of the hose connector (typically configured to receive pressurised fluid (e.g. air) from the fluid (e.g. air) pressurising device, e.g. by way of the pressure chamber). Typically the hose connector is coupled to the housing by way of a (second) coupler which enables (e.g. vibrational) movement of the hose connector relative to the housing to thereby restrict transfer of vibrations from the hose connector to the housing.

Typically at least a portion of the (second) coupler is resilient (e.g. elastic). It may be that the method comprises resiliently (e.g. elastically) deforming at least a portion of the (second) coupler to thereby restrict transfer of vibrations from the hose connector to the housing. It may be that the (second) coupler comprises an elastic sleeve comprising a first portion coupled to the outlet port of the housing and a second end coupled to the hose connector. It may be that the method comprises elastically deforming the elastic sleeve to thereby restrict transfer of vibrations from the hose connector to the housing.

It may be that the fluid (e.g. air) pressurising device comprises a motor. It may be that the method comprises coupling the said motor to the said housing (e.g. by way of the said pressure chamber) by way of the said suspension which enables (e.g. vibrational) movement of the said motor relative to the housing.

It may be that the method comprises (e.g. the or each said suspension) damping (e.g. vibrational) movement of the fluid (e.g. air) pressurising device relative to the housing to thereby restrict transfer of vibrations from the fluid (e.g. air) pressurising device to the housing. It may be that the method comprises damping (e.g. vibrational) movement of the fluid (e.g. air) pressurising device relative to the pressure chamber to thereby restrict transfer of vibrations from the fluid (e.g. air) pressurising device to the housing. It may be that the method comprises damping (e.g. vibrational) movement of the pressure chamber relative to the housing to thereby restrict transfer of vibrations from fluid (e.g. air) pressurising device and/or from the pressure chamber to the housing.

It may be that the method comprises (e.g. the or each said suspension) dissipating energy from (e.g. vibrational) movement of the fluid (e.g. air) pressurising device relative to the housing to thereby restrict transfer of vibrations from the fluid (e.g. air) pressurising device to the housing. It may be that the method comprises dissipating energy from (e.g. vibrational) movement of the fluid (e.g. air) pressurising device relative to the pressure chamber to thereby restrict transfer of vibrations from the fluid (e.g. air) pressurising device to the housing. It may be that the method comprises dissipating energy from (e.g. vibrational) movement of the pressure chamber relative to the housing to thereby restrict transfer of vibrations from the fluid (e.g. air) pressurising device and/or from the pressure chamber to the housing.

It may be that the method comprises (e.g. the or each said suspension) storing energy from (e.g. vibrational) movement of the said fluid (e.g. air) pressurising device relative to the housing to thereby restrict transfer of vibrations from the fluid (e.g. air) pressurising device to the housing. It may be that the method comprises storing energy from (e.g. vibrational) movement of the fluid (e.g. air) pressurising device relative to the pressure chamber to thereby restrict transfer of vibrations from the fluid (e.g. air) pressurising device to the housing. It may be that the method comprises storing energy from (e.g. vibrational) movement of the pressure chamber relative to the housing to thereby restrict transfer of vibrations from the fluid (e.g. air) pressurising device and/or from the pressure chamber to the housing.

It may be that the method comprises resiliently deforming at least a portion of the (or each said) suspension by (e.g. vibrational) movement of the fluid (e.g. air) pressurising device relative to the housing to thereby restrict transfer of vibrations from the fluid (e.g. air) pressurising device to the housing. It may be that the method comprises resiliently deforming at least a portion of the (or each said) suspension by (e.g. vibrational) movement of the fluid (e.g. air) pressurising device relative to the pressure chamber to thereby restrict transfer of vibrations from the fluid (e.g. air) pressurising device to the housing. It may be that the method comprises resiliently deforming at least a portion of the (or each said) suspension by (e.g. vibrational) movement of the pressure chamber relative to the housing to thereby restrict transfer of vibrations from the fluid (e.g. air) pressurising device and/or from the pressure chamber to the housing.

It may be that the method comprises exposing the housing to the environment.

It may be that the method comprises outputting from the housing a heated fluid (e.g. air) flow having a volumetric flow rate of at least 0.001m³/s, more typically at least 0.002m³/s, even more typically at least 0.0025m³/s, in some cases at least 0.01 m3/s, in some cases at least 0.02m³/s, in some cases at least 0.025m³/s.

It may be that the method comprises outputting from the housing a heated fluid (e.g. air) flow having a temperature of at least 30°C, more typically at least 35°C, more typically at least 40°C, typically less than 55°C, preferably between 42°C and 48°C, more preferably between 44°C and 46°C, most preferably at 45°C.

It may be that the method comprises using pressurised fluid (e.g. air) from the pressure chamber to regulate the temperature of a (typically human) patient.

It may be that the method comprises providing pressurised fluid (e.g. air) from the pressure chamber to a patient temperature regulation tool to thereby regulate the temperature of a (typically human) patient. It may be that the patient temperature regulation tool is configured to heat a patient by convection using the pressurised fluid (e.g. air).

It may be that the method comprises transferring pressurised fluid (e.g. air) from the pressure chamber to the patient temperature regulation tool by way of a or the (typically flexible) hose.

It may be that the method comprises mounting the housing adjacent to a (typically human) patient (e.g. the patient's head) or to a bed frame (e.g. a bed frame carrying a human patient). It may be that the method comprises (e.g. vibrationally) moving the housing relative to the bed frame (e.g. by operating (e.g. by resiliently deforming) a resilient mounting coupling the housing to the bed frame) to thereby restrict transfer of vibrations from (e.g. the housing of) the fluid warming apparatus to the bed frame.

It may be that the method comprises pressurising fluid (e.g. air) from the fluid (e.g. air) intake by moving a movable portion (e.g. one or more rotating blades) of the said fluid pressurising device relative to a static portion (e.g. a fluid pressurising device housing) of the fluid pressurising device. It may be that the method comprises operating the said suspension to thereby (e.g. vibrationally) move the static portion of the fluid pressurising device relative to the housing to thereby restrict transfer of vibrations from the said static portion to the housing.

A fourth aspect of the invention provides a method of regulating the temperature of a (typically human) patient (typically during a medical procedure or surgery), the method comprising: providing a fluid (e.g. forced air) warming device within (e.g. encapsulated by) a (typically external) housing. Typically the method further comprises the fluid (e.g. air) pressurising device receiving a fluid (e.g. air) intake. Typically the method further comprises the fluid (e.g. air) pressurising device pressurising fluid (e.g. air) from the fluid (e.g. air) intake (typically by rotation of one or more blades of an air pressurising device (e.g. a fan) provided within the housing about an axis of rotation), typically thereby causing vibrations (typically within the housing). Typically the method further comprises a suspension restricting transfer of (typically said) vibrations (typically from within the housing) to the housing.

Typically the method comprises the said suspension enabling (e.g. vibrational) movement of the fluid (e.g. air) pressurising device relative to the housing to thereby restrict transfer of vibrations from the fluid (e.g. air) pressurising device to the housing. Typically the method further comprises regulating the temperature of the patient using the said pressurised fluid (e.g. air).

Typically the pressurised fluid (e.g. air) is heated. It may be that the pressurised fluid (e.g. air) is heated to a temperature of at least 30°C, more typically at least 35°C, more typically at least 40°C, typically less than 55°C, preferably between 42°C and 48°C, more preferably between 44°C and 46°C, most preferably at 45°C.

It may be that the pressurised fluid (e.g. air) is output from the pressure chamber to a patient temperature regulation tool in order to regulate the temperature of the patient. It may be that the pressurised fluid (e.g. air) output has a volumetric flow rate of at least 0.001 m3/s, more typically at least 0.002m³/s, even more typically at least 0.0025m³/s, in some cases at least 0.01m³/s, in some cases at least 0.02m³/s, in some cases at least 0.025m³/s.

Although various aspects and embodiments of the present invention have been described separately above, any of the aspects and features of the present invention can be used in conjunction with any other aspect, embodiment or feature where appropriate. For example apparatus features may where appropriate be interchanged with method features.

### Description of the Drawings

An example embodiment of the present invention will now be illustrated with reference to the following Figures in which:
Figure 1 is a block diagram showing the rear of a fluid warming apparatus coupled to a perforated blanket by a flexible hose;
Figure 2 shows the fluid warming apparatus of Figure 1, with the housing shown as being transparent to make an inner pressure chamber of the fluid warming apparatus visible;
Figure 3 is a rear schematic view of the pressure chamber of the fluid warming apparatus of Figures 1 and 2;
Figure 4 is a close up schematic view of a suspension mounting which connects the pressure chamber to the housing of the fluid warming apparatus;
Figure 5 is a front schematic view of the pressure chamber, housing and hose of Figures 1-3, the pressure chamber being coupled to the housing by a first coupler and the housing being coupled to the hose connector by a second coupler;
Figure 6 is a front sectional view of an alternative fluid warming apparatus to that shown in Figures 1 to 4, the inner pressure chamber being mechanically coupled to the housing by a suspension having an alternative configuration; and
Figure 7 shows an axial fan which is an alternative to the centrifugal fan of the embodiments of Figures 1 to 6.

### Detailed Description of an Example Embodiment

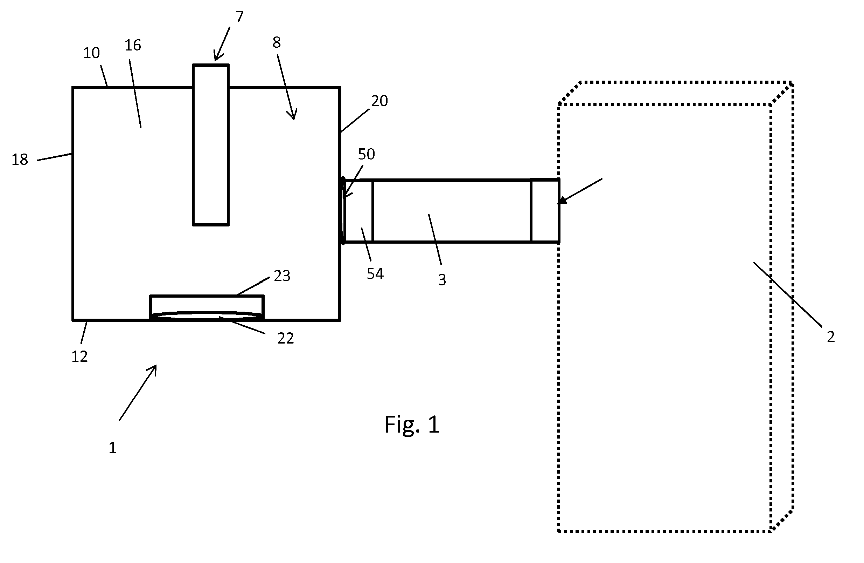

Figure 1 is a block diagram showing the rear of a fluid warming apparatus 1 (typically sealedly) fluidly coupled to a perforated blanket 2 by a flexible hose 3, the perforated blanket 2 being draped over a (typically human) patient (not shown) during a medical procedure or operation (typically when the patient is under a general anaesthetic). Air heated by the fluid warming apparatus 1 is delivered to the blanket 2 through the hose 3. The blanket 2 contains a plurality of perforations (not shown) through which heated air received from the fluid warming apparatus 1 is delivered to the patient to heat the patient by forced convection, thereby preventing the onset of unintentional hypothermia in the patient.

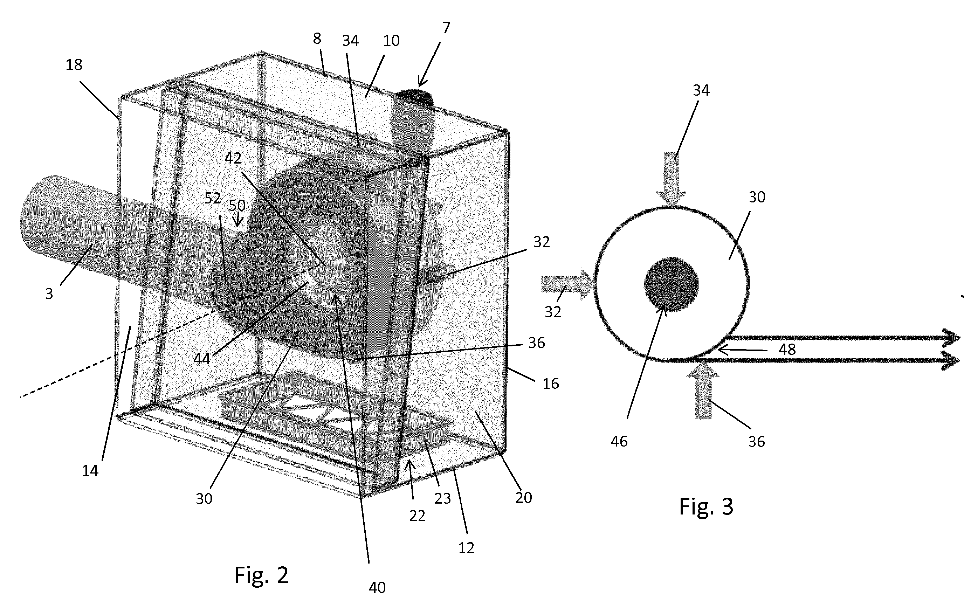

As shown in Figures 1 and 2, the fluid warming apparatus 1 comprises a vertically mounted (by way of a resilient mounting 7) external housing 8 having opposing first (upper) and second (lower) faces 10, 12 extending between opposing third (front) and fourth (rear) faces 14, 16 and between opposing fifth (side) and sixth (side) faces 18, 20. The third (front) and fourth (rear) faces 14, 16 also extend between the fifth (side) and sixth (side) faces 18, 20. The third (front) face 14 is removable so that the inside of the external housing 8 can be accessed for maintenance (although any one or more of the first to sixth faces could be removable for access). The second face 12 of the fluid warming apparatus 1 has an ambient air intake port 22 configured to receive ambient air from the surrounding environment into an inner volume of the housing 8. The air intake port 22 is provided with a filter 23 for filtering the incoming ambient air.

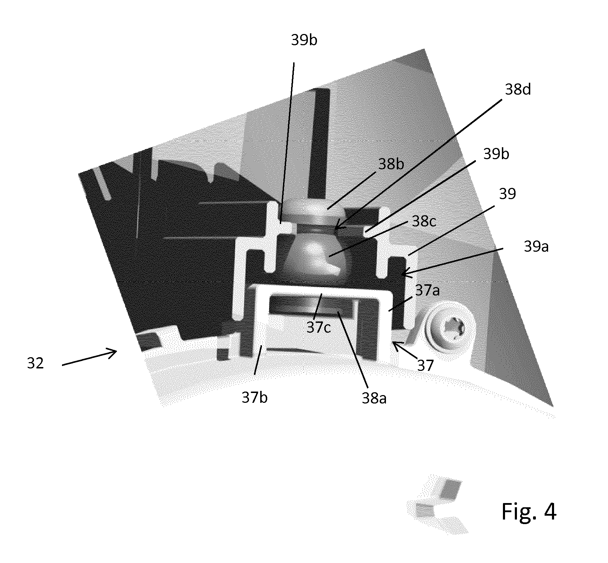

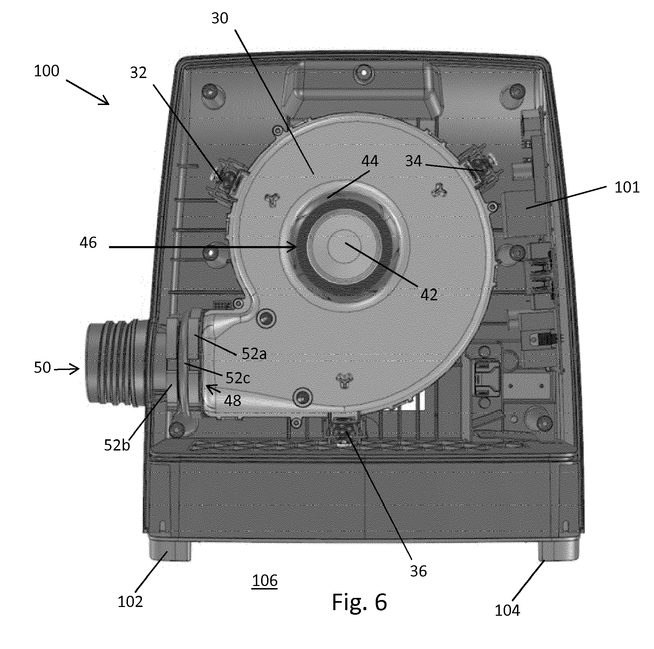

As shown most clearly in Figure 2, within the housing 8 is provided a pressure chamber 30 in the form of an annular volute which is mechanically coupled to an internal surface of the rear face 16 of the housing 8 (although it will be understood that the pressure chamber could alternatively be mounted to any of the other faces of the housing 8) by way of a suspension comprising first, second and third suspension mountings 32, 34 and 36 respectively (the suspension mountings are most clearly shown in Figures 4 and 6, and are not shown in detail in Figure 2). The suspension mountings 32, 34, 36 extend from an outer perimeter of the pressure chamber 30 and are spaced from each other around the said perimeter in a plane perpendicular to the axis of rotation, the second and third suspension mountings 34, 36 extending from upper and lower portions of the perimeter respectively and the first suspension mounting 32 extending from a portion of the perimeter intermediate the second and third mountings 34, 36. This is illustrated most clearly in Figure 3.

The suspension mountings 32, 34, 36 are identical so only suspension mounting 32 will be described in detail, it being understood that suspension mountings 34, 36 comprise the same features. As shown in Figure 4, suspension mounting 32 comprises: an n-shaped bracket 37 having first and second sides 37a, 37b extending outwardly from the outer perimeter of the pressure chamber 30 and a third side 37c extending (and coupling) the first and second sides 37a, 37b; and an elastomeric damper 38, the damper 38 comprising a base 38a, a boss 38b and an intermediate portion 38c extending between the base 38a and the boss 38b. The base 38a (and part of the intermediate portion 38c adjacent the base 38a) of the damper is received by an aperture (not shown) in (and retained by) the third face 37c of the bracket 37, the said aperture having a smaller width than the base 38a. A bracket 39 extends from an inner surface of the rear face 16 of the housing 8 for coupling the housing 8 to the damper 38 (and thus for retaining the pressure chamber 30 on the said rear surface of the housing 8). The bracket 39 comprises a recess 39a into which a portion of the suspension mounting 32 (including a portion of the n-shaped bracket 37 and a portion of the damper 38) is received. The bracket 39 further comprises protrusions 39b which are received by a recess 38d in the damper 38 between the intermediate portion 38c and the boss 38b and the engagement of the protrusions 39b and inner walls of the recess 38d inhibit relative radial movement of the damper 38 and the bracket 39 (in either radial direction). Vibrational movement of the pressure chamber 30 relative to the housing 8 is enabled and damped by movement and/or resilient deformation of the damper 38 relative to the bracket 39. The bracket 39 is however configured to restrict larger movements of the pressure chamber 30 relative to the housing 8 (e.g. beyond the elastic deformation limit of the damper 38), for example in the event of large accelerations (e.g. due to external impacts on the fluid warming apparatus).

In order to design the suspension mountings 32, 34, 36, it is necessary to consider the natural frequency, stroke and mass of the system which is to be damped (in this case, the pressure chamber 30 and its contents) and the environmental conditions (e.g. temperature and humidity) in which it is to be used. At least the following parameters can be tuned to optimise their design: the required lifetime of the suspension mountings 32, 34, 36; the allowed stroke and maximum deflection of the damped system; the stiffness of the damper 38 (or, for embodiments comprising springs, the spring constant of the springs); the required durability of the damper 38; and the number of suspension mountings (which number is dependent on budget and space considerations).

The pressure chamber 30 houses a centrifugal fan 40 which is rigidly mounted to the pressure chamber 30 and which comprises a hub 42 and a plurality of blades 44, proximal ends of the blades 44 being mechanically connected to and extending from the hub 42. The hub 42 houses a motor which, in use, causes the hub 42 (and thus the blades 44) to rotate about an axis of rotation (represented in Figure 2 by a dotted line extending through a centre of the hub 42). The hub 42 is provided within an annulus 46 of the annular pressure chamber 30 and is therefore surrounded by the annular pressure chamber 30 but is not provided within the annular pressure chamber 30. On the other hand, the blades 44 (or at least portions of the blades 44, typically including distal ends thereof remote from the hub 42) are provided within the pressure chamber 30.

It will be understood that, alternatively, a drive shaft may extend into the annulus from a motor positioned elsewhere (other than in the hub) in order to rotate the fan blades.

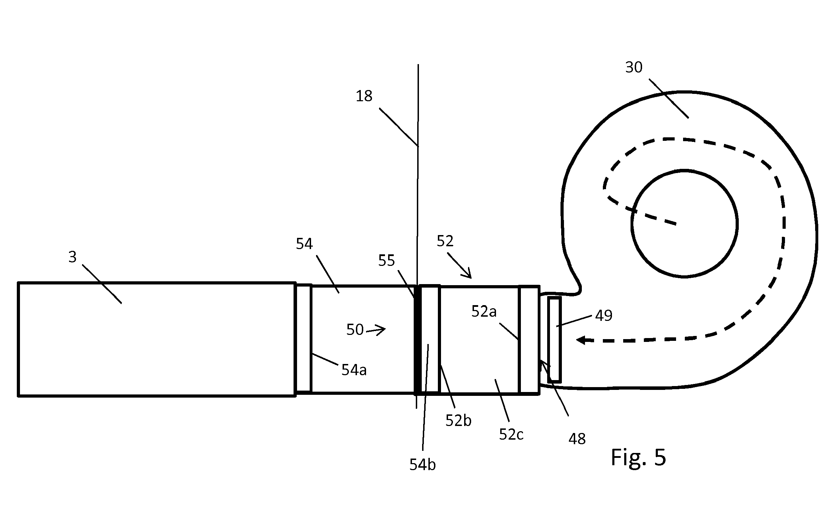

When ambient air is received into the housing 8 through the air intake port 22 and the filter 23, the air within the inner volume of the housing 8 is at underpressure (i.e. at a pressure less than the atmospheric pressure immediately surrounding the housing 8) due to a pressure drop across the filter 23. When the motor causes the hub 42 and thus the blades 44 to rotate, air from the inner volume of the housing 8 is drawn into the annulus 46 of the fan (and ambient air is drawn into the housing 8 through air intake port 22 and the filter 23). Air drawn into the annulus 46 is caused to enter the annular pressure chamber 30 through gaps between adjacent blades 44, and is pressurised (increased in pressure), by rotation of the fan blades 44. The air drawn into the annular pressure chamber 30 flows along an air flow passage extending around the annulus 46 of the pressure chamber 30 (illustrated by the dashed arrow-headed line in Figure 5) to an outlet 48 of the pressure chamber 30. A heater 49 (see Figure 5) is provided at the outlet 48 of the pressure chamber 30 and configured to heat the pressurised air output from the pressure chamber 30 (although the heater may alternatively be provided anywhere along the air flow path between the air intake port 22 of the housing 8 and the hose 3). In this way, a supply of heated, pressurised air is provided in the annular pressure chamber 30. In addition, the ambient air flow through the inner volume of the housing 8 cools electronics provided therein (the electronics are shown in Figure 6 which is described below).

As the motor and the blades 44 of the fan rotate, they vibrate and generate acoustic waves (typically at human audible frequencies) in the pressure chamber 30. In addition, the pressurised air provided in and vibration of the pressure chamber 30 typically flows turbulently, thereby causing vibration of the pressure chamber 30. By providing the blades 44 of the fan (or at least portions thereof) within the pressure chamber 30, the pressure chamber 30 substantially attenuates (e.g. absorbs or reflects) the acoustic waves generated by the blades in use, to thereby reduce the amplitudes of acoustic waves which propagate to the faces of the housing 8. In addition, by coupling the pressure chamber 30 to the housing 8 by way of a suspension 32, 34, 36 which enables movement of the pressure chamber 30 relative to the housing, transfer of vibrations from the pressure chamber 30 to the housing 8 is substantially restricted. This allows the fluid warming apparatus 1 to be mounted closer to the patient (e.g. on a frame of a bed which supports the patient), and even adjacent to the patient's head, without transmitting a significant amount of noise to the patient. In addition, this enables a shorter hose 3 to be employed which improves thermal efficiency as well as the convenience and safety of the system.

In some embodiments, the mounting 7 mounts the fluid warming apparatus 1 to a patient's bed frame, the mounting 7 being resiliently (e.g. elastically) deformable so as to act as a suspension enabling vibrational movement of the housing 8 relative to the bed frame, thereby restricting transfer of vibrations from the housing 8 to the bed frame.

Heated, pressurised air is transferred from the pressure chamber 30 to the hose 3 by way of the outlet 48 of the pressure chamber 30. As most clearly shown in Figure 5 (which omits some elements of the fluid warming apparatus for clarity), heated, pressurised air from the pressure chamber 30 is carried from the outlet 48 of the pressure chamber 30 and out of the housing 8 to the hose 3 through an outlet port 50 of the housing 8 by a coupler 52 extending (at least part of the way) between them. The hose 3 is coupled to the pressure chamber 30 by way of a (typically rigid) hose connector 54. In this embodiment, the hose connector 54 extends through the port 50 from a first end 54a outside the housing 8 to a second end 54b inside the housing 8. The first and second ends 54a, 54b of the hose connector 54 are provided with threaded portions (e.g. it may be that the hose connector 54 is threaded along its length between the first and second ends 54a, 54b). The hose 3 is threadably coupled to the first end 54a of the hose connector 54. The first coupler 52 comprises: a first end 52a having threads which threadably mate with threads at the outlet 48 of the pressure chamber 30 and a second end 52b opposite the first end 52a has threads which threadably mate with threads on the second end 54b of the hose connector 54. Between the first and second ends 52a, 52b of the coupler 52 is provided an elastic sleeve 52c which enables relative (e.g. vibrational) movement of the pressure chamber 30 and the housing 8 and which fluidly couples the hose connector 54 to the outlet of the pressure chamber 30.

By providing the coupler 52 with the elastic sleeve 52c, the transfer of vibrations from the pressure chamber 30 to the housing 3 is further restricted because the propagation of mechanical waves between the pressure chamber 30 and the housing 8 is made less efficient. This is because vibrations of the pressure chamber 30 cause elastic deformation of the sleeve 52c as they propagate away from the pressure chamber 30 towards the housing 8, thereby reducing the amplitude of any remaining vibrations which propagate into the housing by way of the coupler 52. This in turn reduces the vibration and acoustic noise output by the housing 8.

In addition (although it is optional, and indeed can be an alternative to the coupler 52 having an elastic sleeve 52c) a resilient (e.g.) elastic coupler 55 (provided in the form of a sleeve over the hose connector 54) is provided between the hose connector 54 and the housing 8 within the port 50. The elastic coupler 55 extends from the outer perimeter of the hose connector 54 to the housing 8 (i.e. from the hose connector 54 to the inner walls of the port 50), surrounding the hose connector 8 around its perimeter. The elastic coupler 55 enables relative (e.g. vibrational) movement of the hose connector 54 and the housing 55 to thereby further restrict the transfer of vibrations from the hose connector 54 to the housing 8.

Figure 6 is a sectional view of an alternative fluid warming apparatus 100 which is identical to that shown in Figures 1 to 5, but for a different arrangement of the suspension mountings 32, 34, 36 and the provision of feet 102, 104 which support the housing 8 on a mounting surface 106 (typically instead of mounting 7). Suspension mounting 36 is provided in substantially the same place in the arrangement of Figure 6 as in the arrangement of Figures 2-3, and the suspension mountings 32, 34 and 36 are still spaced from each other about the perimeter in a plane perpendicular to the axis of rotation of the fan. However, in a change from the arrangement of Figures 1 to 5, suspension mountings 32, 34 of the arrangement of Figure 6 extend from opposite sides of the perimeter of the pressure chamber 30 at (similar) heights between the axis of rotation and the top of the pressure chamber 30.

For optimal damping, the suspension mountings 32, 34, 36 are provided in the same (typically vertical) plane and the plane comprises (i.e. is aligned with) the centre of gravity of the pressure chamber 30. Providing the suspension mountings 32, 34, 36 in the same plane helps to isolate the vibrations of the pressure chamber 30 from the housing 8. Providing the suspension mountings 32, 34, 36 in the same plane as the centre of gravity of the pressure chamber 30 also helps to limit and control the three dimensional vibrations of the pressure chamber 30. The suspension mountings 32, 34, 36 are also distributed symmetrically about the centre of gravity of the pressure chamber 30 in order to distribute the load evenly among them.

Also shown in Figure 6 are control electronics 101 (e.g. for controlling rotation of the fan and operation of the heater) provided within the housing 8.

Feet 102, 104 can be formed from a resilient elastomer such that they enable (e.g. vibrational) movement of the housing 8 relative to the mounting surface 106. This restricts the transfer of vibrations of the housing 8 to the mounting surface 106.

The other features of the fluid warming apparatus 100 will not be described in detail in view of the fact that they are identical to corresponding features of the embodiment of Figures 1 to 5; identical features have been allocated the same reference numerals (where applicable) as corresponding features of the fluid warming apparatus 1 of Figures 1 to 5. The hose 3 and the blanket 2 have been omitted from Figure 6.

Further variations and modifications may be made within the scope of the invention herein described. For example, it will be understood that the housing 8 does not need to be vertically mounted. It may alternatively be horizontally mounted or mounted at an angle to the horizontal and/or to the vertical.

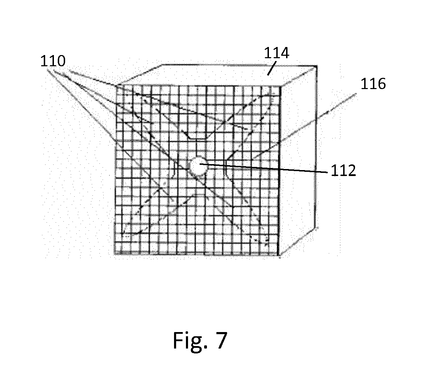

It will also be understood that the fan 40 need not be a centrifugal fan. For example, an axial fan could alternatively be used, such as the one shown in Figure 7. In this case, the fan comprises four blades 110 rotatable about an axis 112 and enclosed within a pressure chamber 114. The pressure chamber 114 is provided with a meshed front face 116 through which pressurised air can be output to a hose as above and typically a meshed rear face through which intake air can be received by the fan. The pressure chamber 114 is typically mounted to the housing 8 by way of suspension mountings as described above.

Although three suspension mountings are provided in each of the embodiments described above, it will be understood that any suitable number of suspension mountings may be provided (e.g. one or more, or more than three) or indeed any other suitable suspension design may be provided which enables (e.g. vibrational) movement of the pressure chamber 30 relative to the housing 8 to restrict transfer of vibrations from the pressure chamber 30 to the housing 8.

It will also be understood that the pressure chamber need not be annular, and need not have a volute (or scroll) shape.

It may be that both the hub and the blades of the fan may be provided within the pressure chamber. Alternatively, the hub may be mounted in the inlet port of the pressure chamber 30 (even when the pressure chamber is not annular and does not have a volute (scroll) shape).

In embodiments in which the housing 8 comprises feet for mounting the housing 8 to a mounting surface, any suitable number of feet may be provided.

The housing may have any suitable shape.

It may be that, instead of being rigidly coupled, the fan 40 is mechanically coupled to the pressure chamber 30 by a suspension (e.g. identical to one or more of the suspension mountings 32, 34, 36 described above) enabling relative (e.g. vibrational) movement of the fan 40 and the pressure chamber 30 to thereby restrict transfer of vibrations of the fan 40 to the housing 8.

It may be that the motor of the fan 40 is mechanically coupled to the pressure chamber 30 or to the housing 8 by a suspension (e.g. identical to one or more of the suspension mountings 32, 34, 36 described above) enabling relative vibrational movement of the motor and the pressure chamber or housing to thereby restrict transfer of vibrations therebetween.

## Claims

1. Forced air warming apparatus for providing air to a patient temperature regulation tool for regulating the temperature of a patient, the forced air warming apparatus comprising: a housing; an air pressurising device provided within the housing, the air pressurising device being configured to receive an air intake and to pressurise air from the air intake; and a suspension configured to restrict transfer to the housing of vibrations caused by the air pressurising device pressurising the said air from the air intake.

2. The forced air warming apparatus according to claim 1 wherein the air pressurising device is mechanically coupled to the housing by way of the said suspension, and wherein the said suspension enables movement of the air pressurising device relative to the housing to thereby restrict transfer of vibrations from the air pressurising device to the housing.

3. The forced air warming apparatus according to claim 1 or claim 2 wherein the air pressurising device is configured to generate pressurised air in a pressure chamber provided within the housing.

4. The forced air warmer according to claim 3 wherein the pressure chamber is mechanically coupled to the housing by way of the said suspension to thereby restrict transfer of vibrations from the pressure chamber to the housing.

5. The forced air warming apparatus according to claim 4 wherein at least a portion of the suspension is configured to damp movement of the pressure chamber relative to the housing to thereby restrict transfer of vibrations from the pressure chamber to the housing.

6. The forced air warmer according to claim 3 wherein the air pressurising device is mechanically coupled to the pressure chamber by way of the said suspension to thereby restrict transfer of vibrations from the air pressurising device to the pressure chamber.

7. The forced air warmer according to any one of claims 3 to 6 wherein the air pressurising device is mechanically coupled to the housing by way of the pressure chamber.

8. The forced air warming apparatus according to any one of claims 3 to 7 wherein the air pressurising device comprises one or more blades rotatable about an axis of rotation to thereby generate the said pressurised air from the air intake, wherein at least a portion of each of the said one or more blades is provided within the pressure chamber.

9. The forced air warming apparatus according to any one of claims 3 to 8 wherein the pressure chamber comprises an air outlet port and the housing comprises an air outlet port, wherein the air outlet port of the pressure chamber is in fluid communication with the air outlet port of the housing by way of a coupler, the said coupler enabling movement of the pressure chamber relative to the housing to thereby restrict transfer of vibrations from the pressure chamber to the housing.

10. The forced air warming apparatus according to any one preceding claim wherein at least a portion of the suspension is resilient.

11. The forced air warming apparatus according to any one preceding claim wherein the said suspension comprises a plurality of suspension mountings, wherein each of the suspension mountings comprises one or more of the group comprising: a damper; a resilient element; a resiliently deformable element; an elastic element; an elastically deformable element; an energy storage element; an elastomeric element; a spring; and a hinge.

12. The forced air warming apparatus according to any one preceding claim further comprising a hose connector, wherein the hose connector is coupled to the housing by way of a coupler which enables movement of the hose connector relative to the housing to thereby restrict transfer of vibrations from the hose connector to the housing.

13. A patient temperature regulation system comprising: a forced air warming apparatus according to any one preceding claim; and a patient temperature regulation tool configured to receive heated air from the forced air warming apparatus by way of a hose.

14. The patient temperature regulation system according to claim 13 wherein the forced air warming apparatus is provided adjacent to a patient or mounted to a bed frame.

15. A method of pressurising air in forced air warming apparatus for providing air to a patient temperature regulation tool for regulating the temperature of a patient, the method comprising: providing an air pressurising device within a housing; the air pressurising device receiving an air intake; the air pressurising device pressurising air from the air intake, thereby causing vibrations; and a suspension restricting transfer of said vibrations to the housing.
